# EUROPEAN PATENT APPLICATION

(11) **EP 2 796 564 A1**
(43) Date of publication of application: **29.10.2014**
(21) Application number: 13165649.8
(22) Date of filing: 26.04.2013
(51) Int. Cl.: C12Q 1/68

(54) **Method for determining hepatotoxicity of a compound.**

(71) Applicant: Rijksinstituut Voor Volksgezondheid En Milieu, 3721 MA Bilthoven (NL)
(72) Inventor: Ven, van der, Leonardus Theodorus Maria, 3720 BA Bilthoven (NL); Driessen, Marja, 3720 BA Bilthoven (NL)
(74) Representative: Habets, Winand

(57) **Abstract**

The invention is in the field of molecular diagnostics. More in particular it provides marker genes for determining the hepatotoxicity of compounds. A method according to the invention employs samples obtained from zebrafish embryos exposed to a potentially hepatotoxic compound and determines expression levels of a number of marker genes in order to distinguish between hepatotoxic compounds and non-hepatotoxic compounds.

## Description

### Field of the invention

The invention is in the field of molecular diagnostics. More in particular it provides marker genes for determining the hepatotoxicity of compounds. A method according to the invention employs samples obtained from zebrafish (Danio rerio) embryos exposed to a potentially hepatotoxic compound and determines expression levels of a number of marker genes in order to distinguish between hepatotoxic compounds and non-hepatotoxic compounds.

### Background of the invention

In mammals, the liver is prone to injury induced by drugs and chemicals as it is the first organ encountered by a compound after absorption from the gastrointestinal tract. Furthermore, the liver is a specialized organ, where xenobiotic biotransformation is carried out due to the location of the liver in the systemic circulation and its physiological structure. Although biotransformation is necessary to inactivate xenobiotics, it may also lead to production of reactive metabolites that are more toxic than the parent compound resulting in liver injury (Jaeschke, H., Gores, G. J., Cederbaum, A. I., Hinson, J. A., Pessayre, D. & Lemasters, J. J. (2002). Mechanisms of hepatotoxicity. Toxicol Sci 65, 166-76.)

Traditionally, animal studies relying on histopathology and clinical chemistry parameters have been the ultimate test for the evaluation of the hepatotoxic properties of chemicals and compounds. Where these *in vivo* animal studies are associated with high costs, long experimental periods, unacceptably large numbers of animals, and questionable predictivity for humans, *in vitro* liver models are a potential alternative to *in vivo* systems in the assessment of drug-induced liver toxicity. A variety of *in vitro* liver models have been developed, which can be divided into 3 major groups including: the isolated cell models, liver slices and isolated organs (Groneberg, D. A., Grosse-Siestrup, C. & Fischer, A. (2002). In vitro models to study hepatotoxicity. Toxicol Pathol 30, 394-9) *In vitro* models are not ideal either due to their lack of cellular interactions and low metabolic capacity. Therefore, there is an urging need to develop and validate other alternative approaches for hepatotoxicity testing.

### Summary of the invention

We surprisingly found that the hepatotoxicity of a compound may be assessed by determining the expression level of a limited number of genes. These marker genes are listed in table 2 and table 3 below. Hence, the invention relates to a method for determining the hepatotoxicity of a compound comprising the steps of:
a. Providing an at least 36 hours post-fertilization (hpf) fish embryo,
b. Contacting the fish embryo with the compound,
c. Allowing the embryo to develop for an amount of time in the presence of the compound,
d. Determining the expression level of genes *zgc:163022, cyp2k19, zgc:158614, zgc:101858, sqrdl, cyp4v7, cyp19a1b, cyp24a1, impdh1b, cyp2aa3, zgc:77778, slc2a912, LOC100003115, LOC100535166, pklr, scg5, LOC100330229, LOC100538043, zgc:154142* and *tcap,*
e. Comparing the expression level of said genes in the embryo with a predetermined reference level and determining the increase or decrease of the expression level relative to the reference value,
wherein a relative increase of the expression level of at least four genes selected from the group consisting of genes *zgc:163022, cyp2k19, zgc:158614, zgc:101858, sqrdl, cyp4v7, cyp19a1b, cyp24a1, impdh1b,* and *cyp2aa3* and/or a relative decrease of at least four genes selected from the group consisting of genes *zgc:77778, slc2a912, LOC100003115, LOC100535166, pklr, scg5, LOC100330229, LOC100538043, zgc:154142* and *tcap* indicates that the compound is hepatotoxic.

### Detailed description of the invention

We found 20 marker genes that may be used in assessing whether a compound is hepatotoxic. These 20 genes may be divided in two groups; first the group of genes that are upregulated in the presence of a hepatotoxic compound (Table 2) and second, a group of genes that is downregulated in the presence of a hepatotoxic compound (Table 3).

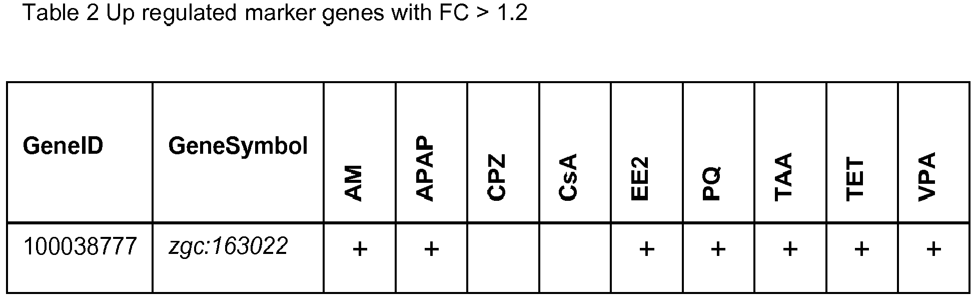

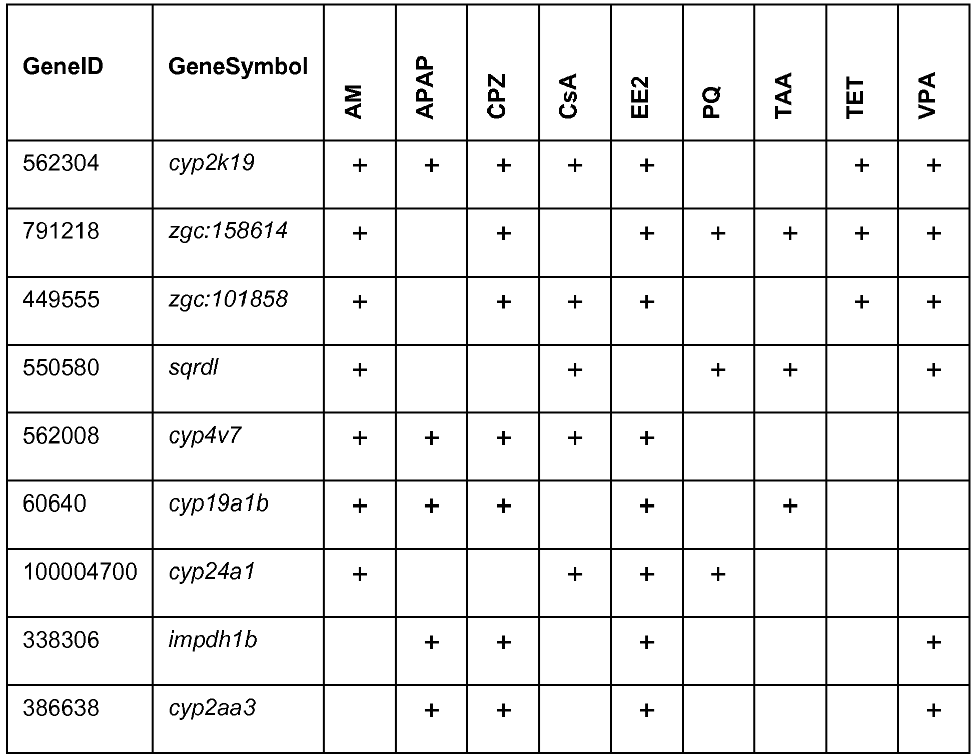

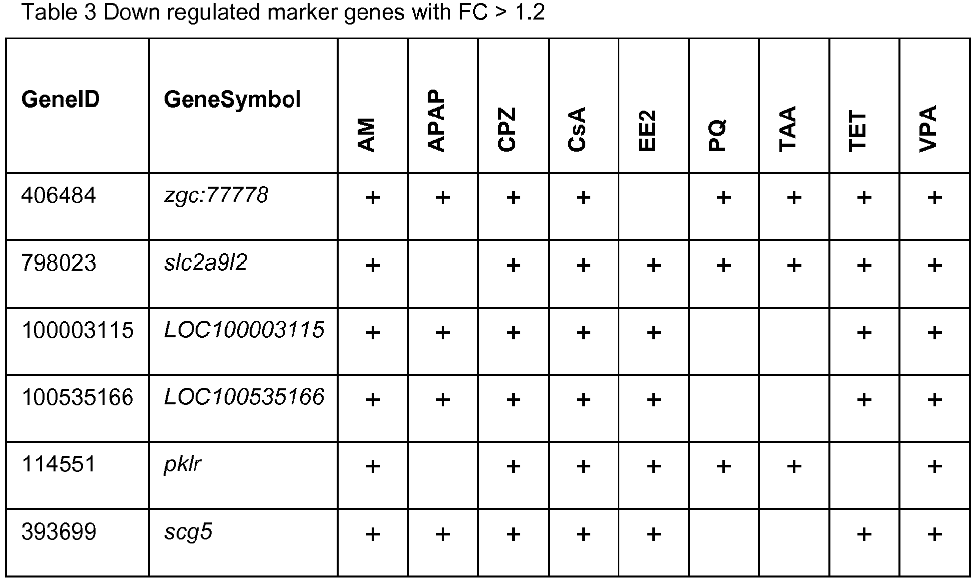

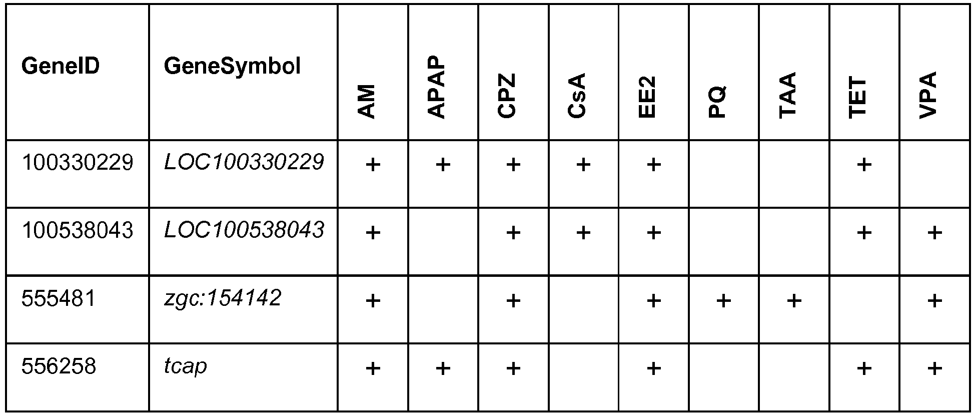

Hence, the invention relates to a method for determining the hepatotoxicity of a compound comprising the steps of:
a. Providing an at least 36 hpf fish embryo,
b. Contacting the fish embryo with the compound,
c. Allowing the embryo to develop for an amount of time in the presence of the compound,
d. Determining the expression level of genes *zgc:163022, cyp2k19, zgc:158614, zgc:101858, sqrdl, cyp4v7, cyp19a1b, cyp24a1, impdh1b, cyp2aa3, zgc:77778, slc2a912, LOC100003115, LOC100535166, pklr, scg5, LOC100330229, LOC100538043, zgc:154142* and *tcap,*
e. Comparing the expression level of said genes in the embryo with a predetermined reference level and determining the increase or decrease of the expression level relative to the reference value,
wherein a relative increase of the expression level of at least four genes selected from the group consisting of genes zgc:163022, cyp2k19, zgc:158614, zgc:101858, sqrdl, cyp4v7, cyp19a1b, cyp24a1, impdh1b, and cyp2aa3 and/or a relative decrease of at least four genes selected from the group consisting of genes zgc:77778, slc2a912, LOC100003115, LOC100535166, pklr, scg5. LOC100330229, LOC100538043, zgc:154142 and tcap indicates that the compound is hepatotoxic.

As used herein, the term "hepatotoxicity" is intended to refer to the property of a compound to interfere with the function of a liver cell to a level that produces microscopically detectable pathological phenotypes. A group of nonlimiting examples of such pathological phenotypes are for instance steatosis, cholestasis, necrosis and vacuolisation.

The term "hepatotoxicity" also refers to injury to the liver that is associated with impaired liver function caused by exposure to a drug or chemical.

We found that the gene expression of 20 genes was significantly altered when exposed to ten different hepatotoxic compounds. When the expression level of these 20 genes was compared to a reference value, it was found that at least 4 genes selected from the group consisting of genes *zgc:163022, cyp2k19, zgc:158614, zgc:101858, sqrdl, cyp4v7, cyp19a1b, cyp24a1, impdh1b,* and *cyp2aa3* were upregulated (table 2). It was also found that at least four genes selected from the group consisting of genes *zgc:77778, slc2a912, LOC100003115, LOC100535166, pklr, scg5, LOC100330229, LOC100538043, zgc:154142* and *tcap* were downregulated (table 3).

Fish embryos start to develop a functional liver about 36 hours after fertilization (hpf). They develop further as an embryo for several days, however, after 120 hours they are considered as fish under the present European Directive. It is therefore preferred to use fish embryos between 36 and 120 hpf. For reasons of sensitivity it is also preferred to use embryos that have a large number of hepatocytes, a completed functional liver is even more preferred. The older the embryo, the shorter the exposure time to the compound can be. We found an optimum between these two considerations by using 72 hpf.fish embryos.

These fish embryos were exposed to the compounds and tested for gene expression. For these purposes fish embryos are preferred since they are easy to culture at low costs and are not considered laboratory animals under European legislation in the stages used in this test (European Commission. Council Directive 86/609/EEC of 24 November 1986 on the approximation of laws, regulations and administrative provisions of the Member States regarding the protection of animals used for experimental and other scientific purposes. Official Journal L 358 , 18/12/1986. 1986: P. 0001 - 28.). On 22 September 2010 the EU adopted Directive 2010/63/EU which updates and replaces the 1986 Directive 86/609/EEC on the protection of animals used for scientific purposes.

The method according to the invention may however be employed with other embryos without deviating from the inventive concept thereof. In a preferred embodiment, the fish is a zebrafish, however other fish species may also be used, for instance medaka, carp or fathead minnow.

The fish embryos were contacted with the compounds in solution. It should be noted that only sub-toxic concentrations of the compounds were used. The term sub-toxic in this context is used as to indicthat the concentrations used were not toxic for the entire organism, that is concentrations that do not induce general toxicity. Such sub-toxic concentrations can easily be determined by a skilled person using his routine skills, for instance by incubating the embryos with limiting dilutions of the compound in culture solution.

Next, the embryo is allowed to develop in the presence of the compound for a certain period of time. For each individual system, the optimal exposure time may be determined, in our experimental setting, a 12 - 96 hour exposure was found satisfactory, however exposure times of 24 - 72 hours are preferred, such as 36 - 60 hours. In the experiments described herein, an exposure time of 48 hours was found most convenient.

After the exposure, total RNA may be isolated from the embryo. This may be performed in any way known in the art, for instance by using a standardized kit such as the RNeasy mini kit from Qiagen or using the method as described by De Jong et al (de Jong M, Rauwerda H, Bruning O, Verkooijen J, Spaink H, Breit T. RNA isolation method for single embryo transcriptome analysis in zebrafish. BMC Research Notes. 2010;3:73). With both methods RNA of good quality can be obtained.

Total RNA may be used to determine the expression levels of any of the 20 genes disclosed herein. This may be done preferably by quantitative real-time PCR or microarray analysis, given costs and work effort required under current state of the art; but it can also be done by or other methods such as Northern blot analysis, Whole Transcriptome Shotgun Sequencing (RNA-seq), or serial analysis of gene expression (SAGE).

In an alternative embodiment, transgenic fish such as zebrafish may be used. In such a transgenic fish or fish embryo a reporter gene is placed under the control of the promoter of the gene of interest. The reporter gene can then be used as a read-out whether the compound is a hepatotoxic compound. The procedures for preparing transgenic fish is detailed in Fisher et al., Nature Protocols 1: 1297 - 1305 (2006). Once a transgenic fish carrying a reporter construct for one of the genes of interest as disclosed herein, other transgenic fish carrying multiple reporter constructs may be obtained by regular breeding. Transgenic fish may then be employed to produce transgenic embryos. Transgenic embryos may be used in a method according to the invention wherein the expression of a gene may be measured by directly determining the amount of the product of the reporter gene, such as a luciferase gene or a fluorescent protein (FP) such as Green Fluorescent Protein (GFP). This obviates the need for extracting RNA after step C of the method as disclosed above.

Fish or fish embryos carrying more than one trangenic construct, may be advantageously be used in a method according to the invention. In such fish or fish embryos, the different promoters of the marker genes may be functionally coupled to different reporter genes so that the expression of several markers may be monitored simultaneously by detecting different signals. For example, the promoter of marker gene A may be coupled to GFP whereas the promoter of gene B is coupled to luciferase. The signals geberated by GFP and luciferase may be read simultaneously.

Reference values for the expression levels of the genes when not exposed to a hepatotoxic compound may be obtained by performing a method as described above without contacting the embryo with a compound. Reference values may also be obtained by performing the method according to the invention with a known non-hepatotoxic compound. It is preferred to use a reference value obtained without contacting the embryo with a compound.

In a preferred embodiment, the change in expression level of the genes identified herein is at least 20% of the reference value. This is a difference that can easily be detected by the above mentioned methods for determining gene expression and therefore allows for robust measurement.

In another preferred embodiment, the decreased or increased expression level of the genes identified herein is at least 25% of the reference value. This is a difference that can easily be detected by the above mentioned methods for determining gene expression and therefore allows for robust measurement.

In general it may be stated that the reliability and robustness of the method according to the invention increases with an increasing change in expression level. An increase or decrease of 50% of the reference value is therefore preferred.

In another preferred embodiment, the method according to the invention is performed wherein a 72 hpf fish embryo is provided.

The results obtained by the method were found to be even more accurate when the subsequent method steps a) to e) were repeated and wherein the increase or decrease of said gene expression was calculated as an average of the independent repeats. The invention therefore relates to a method as described above, wherein the subsequent method steps a) to e) are repeated and wherein the increase or decrease of said gene expression is an average of the independent repeats.

The zebrafish embryo has emerged as a powerful model for the evaluation of drugs and chemicals. Several features make this zebrafish model attractive, among which is the ability to maintain large stocks of the adult zebrafish and zebrafish embryo because of their small size and high fecundity. These advantages enable them to be used in a high-throughput screening setting. Additionally, direct observation and experimental manipulation of tissue and organs is relatively simple in the embryo due to the transparency and the rapid ex utero development. Zebrafish embryogenesis is essentially complete by 72 hours post fertilization at which time the liver is perfused with blood and is fully functional.

In this study, the whole zebrafish embryo model successfully detected hepatotoxicants with higher specificity than the HepG2 cells. The use of toxicogenomics in the zebrafish embryo is a promising approach to unravel these potential mechanisms by studying the effect of pharmaceuticals and chemicals on differential gene expression patterns implicated for toxicity.

### Examples

### Example 1: Materials.

All tested chemicals were purchased from Sigma Aldrich (Zwijndrecht, the Netherlands), and included acetaminophen or paracetamol (N-actyl-para-aminophenol; APAP, CAS no.103-90-2), paraquat (1,1'-Dimethyl-4,4'-bipyridinium dichloride; PQ, CAS no.1910-42-5), thioacetamide((CH3-C(S)NH2); TA, CAS no.62-55-5), amiodarone hydrochloride (2-butyl-3-benzofuranyl-4-[2-(diethylamino)ethoxy]-3,5-diiodophenyl ketone hydrochloride; AM, CAS no. 19774-82-4), valproic acid (2-propylpentanoic acid sodium; VPA, CAS no.1069-66-5), tetracycline (TET, CAS no.64-75-5), cyclosporine A (CsA, CAS no.59865-13-3),17α- ethinylestradiol (17α-Ethinyl-1,3,5(10)-estratriene-3,17β-dioi; EE2, CAS no.57-63-6), chlorpromazine (2-Chloro-10-(3-dimethylaminopropyl)phenothiazine hydrochloride; CPZ, CAS no.69-09-0), and tricaine methansulfonate (MS-222, CAS no. 886-86-2). Dimethylsulfoxide (DMSO, CAS no. 67-68-5) was ordered from Fisher-Scientific. The RNeasy MinElute Cleanup kit (Cat. No. 74204) and the QIAzol Lysis reagent (Cat. No. 79306) were obtained from Qiagen Benelux B.V. (Venlo, the Netherlands). Phase-lock Gel Heavy (Cat. No. 2302870) and the metal micro pestle (P985.1) were purchased from VWR International B.V. (Amsterdam, the Netherlands).

### Example 2: Fish maintenance and spawning.

Wild-type zebrafish (Danio Rerio) were originally obtained as commercially bred Singapore import (Ruinemans Aquarium BV, Montfoort, The Netherlands), which were maintained and bred in our facilities for more than 5 generations. Egg production was optimized by separation of the male and female fish before spawning, and female zebrafish were fed only thawed *Artemia Naupli* prior to spawning. Egg predation was prevented by using a breeding tank, in which male and female zebrafish were paired in a 2:2 ratio before spawning. Spawning was triggered by light and was usually completed within 30 minutes. After spawning, the eggs were collected using a glass siphon. Fertilized batches were rinsed several times in Dutch Standard Water (DSW; demineralized water supplemented with NaHCO₃ (100 mg/l), KHCO₃ (20 mg/l), CaCl₂·2H₂O (200 mg/l), and MgSO₄·7H₂O (180 mg/l)) and then aerated for 24 h at 27 °C to remove debris. After rinsing, the eggs were pooled and placed in a petri dish in an incubator at 26.5 ± 1°C with a photoperiod of 14 hours light: 10 hours dark for 72 hours. Hereafter, hatched embryos were selected and transferred to a 48-well plate, each well receiving 5 embryos, for use in the hepatotoxicity test.

### Example 3: Zebrafish embryo hepatotoxicity test.

The hatched zebrafish embryos (ZFE) were exposed to a set of human model hepatotoxicants (Table 1). Each well contained 1 ml of test medium and each 48-well plate was placed back in the incubator for 48 hrs of exposure, which is completed at 120 hours post fertilization (hpf). Thereafter, zebrafish embryos were evaluated under a Leica Labovert FS microscope for anomalies or snap frozen in liquid nitrogen to be used for microarray analysis.

**Table 1. Model compounds and their concentrations used during the zebrafish embryo hepatotoxicity test**

| **Compound** | **Test concentration (µM)** | **Vehicle control** |
|---|---|---|
| Amiodarone (AMD) | 10 | DMSO |
| Acetaminophen (APAP | 660 | DMSO |
| Chlorpromazine (CPZ) | 3 | DMSO |
| Cyclosporine (CsA) | 6 | DMSO |
| 17 alpha-ethinylestradiol (EE2) | 3.5 | DMSO |
| Paraquat (PQ) | 3000 | DSW |
| Thioacetamide (TAA) | 10000 | DSW |
| Tetracycline (TET) | 200 | DMSO |
| Valproic acid (VPA) | 600 | DSW |
| D-Mannitol (DM) | 3000 | DSW |
| Lithium carbonate (Li₂CO₃) | 1100 | DSW |

### Example 4: Histology.

For histopathology, ZFE were sampled from the three highest concentrations without mortality or teratogenicity from the concentration range finding study. Whole ZFE were fixed in 4% paraformaldehyde for 24 hrs, then transferred and stored in 70% ethanol until use. Samples were first embedded in a specially designed 1% agarose mold for adequate positioning of the embryos (Tsao-Wu et al., Biotechniques 25: 614-618 (1998); Sabaliauskas et al., Methods 39: 246-254, (2006)), and then transferred to paraffin, whereafter 4 µm sections were routinely stained with hematoxylin and eosin (H&E) and covered with a glass coverslip. Additional cryosections from AM, VPA and TET were stained with Oil Red O to determine fatty droplet accumulation. Approximately 12 replicates were included per compound.

### Example 5: RNA isolation and processing.

For microarray analysis, 15 embryos per samples were collected and snap frozen in liquid nitrogen. For all compounds, 4 to 5 biological replicate samples were used for transcriptomics analysis. Total RNA was isolated using the MinElute Cleanup Kit according to the protocol of De Jong et al. (de Jong, M., Rauwerda, H., Bruning, O., Verkooijen, J., Spaink, H. P. & Breit, T. M. (2010). RNA isolation method for single embryo transcriptome analysis in zebrafish. BMC Res Notes 3, 73). RNA concentration was measured on the NanoDrop spectrophotometer (ND-1000, NanoDrop Technologies Inc., Wilmington, DE) and RNA integrity was assessed by automated gel electrophoresis using the RNA 6000 Nano Chip Kit (Bioanalyzer 2100, Agilent technologies, Amstelveen, The Netherlands). Total RNA samples with an RNA Integrity Number (RIN) > 7 were used for further analysis.

### Example 6: GeneChip hybridization.

Technical handling of the microarrays was performed at ServiceXS B.V. (Leiden, the Netherlands). The concentration and the quality of the RNA samples were assessed using the Nanodrop (ND1000; NanoDrop technologies, Wilmington, DE) and BioAnalyzer (Agilent Technologies, Amstelveen, the Netherlands). The Ambion WT Expression kit (#4411974) was used to synthesize labeled sense stranded cDNA starting from 100ng total RNA. The minimal yield of the cRNA product is 10 µg. The Affymetrix Terminal Labeling Kit (901524) was used to perform the fragmentation and terminal labeling step using 5.5 µg of the ss cDNA. The concentration and the quality of the cRNA - and fragmented ss cDNA were assessed using the Nanodrop, BioAnalyzer and MultiNA. Quality control of the fragment length is necessary in order to assess if cRNA fragments of the correct size have been formed. A total amount of 2.9 µg fragmented ss cDNA was finally utilized for the hybridization on the Affymetrix Zebrafish ST array plate. The (#901622) WT for GeneTitan Hybridization, Wash and Stain Kit was used for the hybridization, washing, staining and scanning of the chips. The entire experimental procedure was carried out according to ServiceXS Standard Operating Procedures (SOPs) which have been validated for use with the Affymetrix kits and GeneChips and are completely compatible with the Affymetrix protocols. The software program Affymetrix GeneChip Command Console (v3.2) was used to operate the Affymetrix fluidics stations which process the washing and staining of the cartridges fully automated. After scanning, the array images (DAT files) as well as the visual inspection of the correct alignment of the grid were inspected using the program Affymetrix Command Console Viewer software.

### Example 7: Data processing and quality control.

Affymetrix Cell Intensity Files (*.cel) were normalized using the Robust Multichip Average (RMA) algorithm12 and the MBNI custom CDF version 15 (http://brainarray.mnbi.medumich.edu/Brainarray/Database/CustomCDF). The quality of micro array images was inspected visually (http://arrayanalysis.org, BiGCaT Maastricht University). Four out of 192 arrays were eliminated from our analysis because they did not meet the quality criteria. Affymetrix internal controls ID were not used in further analyses, leaving a total of 23,758 probe sets corresponding to unique Entrez GeneIDs. RMA normalized data were transformed to Log2 using R (version 2.15.0) for further analysis.

### Example 8: Compound-induced gene expression.

Log2-transformed data was corrected with the median of the vehicle controls. Gene expression data were analysed by one-way ANOVA with an FDR<0.05 and a post-hoc test (two sided t-test, p ≤0.002225342 corresponded to FDR < 0.05) was applied between compound exposed and vehicle control embryos to examine the gene expression responses compared to control. Genes with an absolute fold change (FG) > 1.2 were considered to be significantly differentially expressed.

## Claims

1. Method for determining the hepatotoxicity of a compound comprising the steps of:
a. Providing an at least 36 hours post fertilization (hpf) fish embryo,
b. Contacting the fish embryo with the compound,
c. Allowing the embryo to develop for an amount of time in the presence of the compound,
d. Determining the expression level of genes *zgc:163022, cyp2k19, zgc:158614, zgc:101858, sqrdl, cyp4v7, cyp19a1b, cyp24a1, impdh1b, cyp2aa3, zgc:77778, slc2a912, LOC100003115, LOC100535166, pklr, scg5, LOC100330229, LOC100538043, zgc:154142* and *tcap,*
e. Comparing the expression level of said genes in the embryo with a predetermined reference level and determining the increase or decrease of the expression level relative to the reference value,
wherein a relative increase of the expression level of at least four genes selected from the group consisting of genes *zgc:163022, cyp2k19, zgc:158614, zgc:101858, sqrdl, cyp4v7, cyp19a1b, cyp24a1, impdh1b,* and *cyp2aa3* and/or a relative decrease of at least four genes selected from the group consisting of genes *zgc:77778, slc2a912, LOC100003115, LOC100535166, pklr, scg5, LOC100330229, LOC100538043, zgc:154142* and *tcap* indicates that the compound is hepatotoxic.

2. Method according to claim 1 wherein the relative increase or decrease of the expression level of genes is at least 20%.

3. Method according to claims 1 or 2 wherein an at least 72 hpf fish embryo is provided.

4. Method according to claims 1 - 3 wherein the fish is a zebrafish (*Danio rerio).*

5. Method according to claims 1 - 4 wherein the embryo is allowed to develop for an amount of time between 12 and 96 hours in the presence of the compound.

6. Method according to claim 5 wherein the amount of time is about 48 hours.

7. Method according to claims 1 - 6 wherein the compound is contacted with the embryo in a concentration just below its toxic level.

8. Method according to claims 1 - 7 wherein RNA is extracted from the embryo after step c) and wherein this extracted RNA is used to determine the expression level of the genes.

9. Method according to claims 1 - 7 wherein the expression level of the at least 4 genes is determined in transgenic zebrafish embryos or mature zebrafish.
